# EUROPEAN PATENT SPECIFICATION

(11) **EP 4 115 838 B1**
(45) Date of publication and mention of the grant of the patent: **12.06.2024**
(21) Application number: 21184663.9
(22) Date of filing: 09.07.2021
(51) Int. Cl.: A61B 34/20

(54) **VIRTUAL 6-DOF TRACKER FOR SURGICAL NAVIGATION**
VIRTUELLER 6-DOF-TRACKER FÜR CHIRURGISCHE NAVIGATION
SUIVEUR VIRTUEL 6-DOF POUR LA NAVIGATION CHIRURGICALE

(43) Date of publication of application: 11.01.2023
(73) Proprietor: Stryker European Operations Limited, Carrigtwohill, Co. Cork T45 HX08 (IE)
(72) Inventor: SCHOEPP, Hans, 79110 Freiburg (DE); Dr. HERRMANN, Florian, 77963 Schwanau (DE)
(74) Representative: Röthinger, Rainer

(56) References cited:
- WO-A1-2021/076560
- US-A1- 2014 073 914
- US-A1- 2019 357 986

## Description

### Technical Field

The present disclosure generally relates to surgical navigation. In particular, a method for use in surgical navigation, a computing system, a surgical navigation system and a computer program product are described.

### Background

In surgical navigation, a pose (i.e., at least one of a position and an orientation) of a surgical instrument relative to an anatomical object (e.g., a bone such as a vertebra, an organ such as the brain etc.), may be tracked. A surgeon may be provided with a view in which the pose of the surgical instrument is indicated relative to patient image data. Such a view is be especially advantageous in case the anatomical object is not visible to the surgeon, for example in case of minimally invasive surgical procedures.

In order to track the pose of the surgical instrument relative to the anatomical object, an optical tracking system may be provided, which is configured to identify one or more (e.g., passive or active) optical tracking markers in images acquired intraoperatively by a (e.g., stereo) camera of the optical tracking system. Alternatively or additionally, an electromagnetic tracking system may be provided, which is configured to determine poses of one or more electromagnetic sensors relative to an electromagnetic field generator of the electromagnetic tracking system. Some of the optical tracking markers or electromagnetic sensors may be located in a fixed pose relative to the anatomical object and may be used as a patient tracker. Some of the optical markers or electromagnetic sensors may be arranged on the surgical instrument and may be used as an instrument tracker. Such an approach allows determining a spatial relationship between the patient tracker and the instrument tracker. In order to display the pose of the surgical instrument relative to the patient image data, a transformation or registration between a coordinate system of the preoperative image data and a tracked pose of the patient tracker may be required, as is known in the art.

In current surgical navigation setups, arrangements comprising a plurality of optical markers or a plurality of electromagnetic coils are used, each arrangement forming a single tracker allowing determination of a pose of the single tracker in six degrees of freedom, DOF. Usually, such arrangements comprise at least three optical markers arranged asymmetrically or at least two electromagnetic coils arranged non-parallel to one another.

The aforementioned 6-DOF trackers may take up much space in some surgical scenarios. For instance, in minimally invasive spinal surgery a patient tracker may be attached on a vertebra into which pedicle screws are to be inserted. Although this may lead to an accurate tracking of the vertebra, the patient tracker may take up space otherwise usable by the surgeon for performing the surgical procedure.

Trackers in current solutions must be rigidly attached relative to the anatomical object or the surgical instrument. Any change in position or orientation of the tracker relative to the object to which the tracker is attached (e.g., the anatomical object) is to be avoided. Namely, such changes may lead to an inaccurate determination of a current pose of the object based on the tracked pose of the attached tracker. Providing a rigid connection between the tracker and the anatomical object may be cumbersome, for example in case of small anatomical objects or limited operating space (e.g., in minimally invasive surgery). Maintaining such a rigid connection during a surgical procedure may impose movement restrictions on a surgeon and increase pressure on the surgeon and other involved clinical personnel, potentially negatively affecting the clinical outcome.

WO 2021/076560 A1 discloses methods, non-transitory computer readable media, and surgical computing devices that track a first position of an external tracker attached externally to a patient based on a tracking array rigidly attached to the external tracker. An electromagnetic field generated by one or more electromagnetic transmitting coils of the external tracker is received by one or more electromagnetic receiving coils of an internal tracker attached to anatomy of the patient. Field data is then generated by the internal tracker based on the received electromagnetic field. A second position of the internal tracker is generated from the field data. The second position is relative to the first position. A third position of the internal tracker is subsequently determined based on the first position and the second position. The third position is within a global tracking system associated with an operating environment.

### Summary

There is a need for a technique that solves one or more of the aforementioned or other problems. The invention is defined in the independent claims.

According to a first aspect, a method for use in surgical navigation is provided. The method is performed by a computing system and comprises obtaining first tracking data for a first tracker in a tracking coordinate system, wherein the first tracker is associated with an anatomical object, and determining, based on the first tracking data, a first pose of the first tracker (e.g., in exactly or only four degrees of freedom, DOF). The method further comprises obtaining second tracking data for a second tracker in a (e.g., the) tracking coordinate system, wherein the second tracker is associated with the anatomical object, and determining, based on the second tracking data, a second pose of the second tracker (e.g., in exactly or only four DOF). The method further comprises determining, based on the first pose and the second pose, a third pose of a virtual tracker in six DOF, the virtual tracker having a fixed spatial relationship relative to the anatomical object in the six DOF of the third pose.

The first tracking data may be obtained from one or more (e.g., optical or electromagnetic) tracking systems (e.g., associated with the tracking coordinate system of the first tracking data). The second tracking data may be obtained from (e.g., the) one or more tracking systems (e.g., associated with the tracking coordinate system of the second tracking data). The first tracking data and the second tracking data may be obtained from the same one or more tracking systems. The coordinate systems of the first and the second tracking data may correspond to one another. The first pose may be determined in the same coordinate system as the second pose. The third pose may be determined in the same coordinate system as at least one of the first pose and the second pose.

The method may further comprise determining or obtaining a transformation between (i) a fourth pose of the anatomical object (e.g., in six DOF) in an image coordinate system of image data of the anatomical object and (ii) the third pose (e.g., in the tracking coordinate system). The transformation may be determined by matching positions acquired relative to the anatomical object by tracking a registration instrument to the image data. A point matching, point cloud matching or surface matching algorithm may be used for this matching operation. Alternative variants of determining the transformation may be used, one of which being described further below. The image data may not be obtained from the (e.g., optical) tracking system. The image data may comprise at least one of preoperative image data and intraoperative image data. The image data may be obtained from an image acquisition device such as a computed tomography, CT, scanner, a magnetic resonance, MR, scanner or an X-ray imaging device.

The method may further comprise determining a fifth pose of the anatomical object in six DOF (e.g., in the tracking coordinate system) based on the third pose and the transformation. The method may further comprise obtaining third tracking data for a third tracker in a (e.g., the) tracking system, determining a pose of a surgical having a fixed spatial position relative to the third tracker, and determining a visualization of the pose of the surgical instrument relative to the anatomical object. The visualization may be displayed.

The first tracker may be arranged in a fixed spatial relationship relative to the anatomical object in only (e.g., the) four or five DOF. The second tracker may be arranged in a fixed spatial relationship relative to the anatomical object in only (e.g., the) four or five DOF. The determined first pose may define a fixed spatial relationship of the first tracker relative to the anatomical object in only (e.g., the) four or five DOF of the first pose. The determined second pose may define a fixed spatial relationship of the second tracker relative to the anatomical object in only (e.g., the) four or five DOF of the second pose.

The four or five DOF (e.g., of at least one of the first pose and the second pose) must comprise two translational DOF and two rotational DOF.

A first of the two translational DOF and a first of the two rotational DOF may be associated with a first trajectory. A second of the two translational DOF and a second of the two rotational DOF may be associated with a second trajectory. The second trajectory may be at least one of different from, non-parallel to and orthogonal to the first trajectory.

Determining the third pose of the virtual tracker may comprise determining, based on one of the first pose and the second pose, a third trajectory defined by the first or second tracker associated with the one of the first pose and the second pose, and determining the third pose of the virtual tracker further based on the third trajectory.

The third trajectory may be associated with at least one DOF excluded by the four or five DOF of the one of the first pose and the second pose. The third trajectory may be associated with at least one DOF in which the first or second tracker is not spatially fixed relative to the anatomical object. The at least one DOF may comprise at least one of exactly one translational DOF and exactly one rotational DOF. The third trajectory may be at least one of different from, non-parallel to and orthogonal to one or both of the first trajectory and the second trajectory.

Determining the third pose of the virtual tracker may comprise determining, based on the first pose, a first instance of the third trajectory. Determining the third pose of the virtual tracker may further comprise determining, based on the second pose, a second instance of the third trajectory. The second instance of the third trajectory may be non-parallel (e.g., skew) relative to the first instance of the third trajectory.

Determining the third pose of the virtual tracker may further comprise determining, based on the first instance of the third trajectory and the second instance of the third trajectory, a virtual coordinate system (e.g., in or relative to the tracking coordinate system). The virtual coordinate system may have a predefined spatial relationship relative to the third pose of the virtual tracker. The virtual coordinate system may correspond to or define the third pose of the virtual tracker.

Determining the virtual coordinate system may comprise determining a first directional vector of the first instance of the third trajectory, determining a second directional vector of the second instance of the third trajectory, and defining a coordinate axis of the virtual coordinate system to lie within a plane spanned by the first directional vector and the second directional vector.

Determining the virtual coordinate system may comprise defining a coordinate axis of the virtual coordinate system to coincide with one of the first instance of the third trajectory and the second instance of the third trajectory.

Determining the virtual coordinate system may comprise determining a direction of a smallest distance between the first instance of the third trajectory and the second instance of the third trajectory, and defining a coordinate axis of the virtual coordinate system to coincide with the direction.

Determining the transformation may comprise detecting an alignment trajectory in the image data, the alignment trajectory having a known spatial relationship relative to at least one of the first tracker and the second tracker, and matching the detected alignment trajectory to the pose of the at least one of the first tracker and the second tracker, using the known spatial relationship. Determining the transformation may comprise detecting two alignment trajectories in the image data, each alignment trajectory having a known spatial relationship relative to one of the first tracker and the second tracker, and matching each detected alignment trajectory to the pose of the one of the first tracker and the second tracker, using the known spatial relationship. The known spatial relationship may define that a first of the alignment trajectories is parallel to or coincides with the third trajectory determined based on the first pose and the first of the alignment trajectories may be matched to the third trajectory determined based on the first pose. The known spatial relationship may define that a second of the alignment trajectories is parallel to or coincides with the third trajectory determined based on the second pose and the second of the alignment trajectories may be matched to the third trajectory determined based on the second pose.

At least one of the first tracker and the second tracker may be movably arranged along an (e.g., the) alignment trajectory having a fixed spatial relationship relative to the anatomical object. The at least one of the first tracker and the second tracker may be translationally movable along the alignment axis. Alternatively or additionally, the at least one of the first tracker and the second tracker may be rotationally movable around the alignment axis.

The at least one of the first tracker and the second tracker may be mechanically coupled to one or more elements chosen from: (i) a linear member arranged in a fixed spatial relationship relative to the anatomical object, wherein the alignment trajectory corresponds to or is parallel to a longitudinal axis of the linear member; (ii) a cannulated implant, the cannulated implant arranged in a fixed spatial relationship relative to the anatomical object, wherein the alignment trajectory corresponds to or is parallel to a longitudinal axis of a cannulation within the cannulated implant; (iii) a surgical instrument, the surgical instrument arranged in a fixed spatial relationship relative to the anatomical object, wherein the alignment trajectory corresponds to or is parallel to a longitudinal axis of a cannulation or tracker receptacle within the surgical instrument; and (iv) a linear hole in the anatomical object, wherein the alignment trajectory corresponds to or is parallel to a longitudinal axis of the linear hole.

The first tracking data may be indicative of a tracked pose of the first tracker in five or six DOF. Determining the first pose may comprise selectively disregarding one or two of the DOF of the tracked pose of the first tracker to obtain the first pose in the exactly four DOF. Alternative or additionally, the second tracking data may be indicative of a tracked pose of the second tracker in five or six DOF. Determining the second pose may comprise selectively disregarding one or two of the DOF of the tracked pose of the second tracker to obtain the second pose in the exactly four DOF.

At least one of the first tracker and the second tracker may be a 3-DOF, 4-DOF or 5-DOF tracker comprising a coil (e.g., extending around a central axis), wherein the coil is configured to be tracked by an electromagnetic tracking system. The central axis may be parallel to or coincide with the alignment trajectory. Alternatively or additionally, at least one of the first tracker and the second tracker may be a 3-DOF, 4-DOF or 5-DOF tracker comprising one, two or more optical tracking markers defining a line in space, wherein the markers are configured to be tracked by an optical tracking system. The line in space may be parallel to or coincide with the alignment trajectory.

The anatomical object may comprise a part of a body of a (e.g., human or animal) patient. The anatomical object may comprise or be a vertebra of a spine of a human patient.

According to a second aspect, a computing system is provided. The computing system comprises at least one processor and at least one memory, the at least one memory storing instructions which, when executed by the at least one processor, configure the at least one processor to perform the method according to the first aspect. The instructions may, when executed by the processor, configure the at least one processor to obtain first tracking data for a first tracker in a tracking coordinate system, wherein the first tracker is associated with an anatomical object, determine, based on the first tracking data, a first pose of the first tracker (e.g., in exactly four degrees of freedom, DOF), obtain second tracking data for a second tracker in a (e.g., the) tracking coordinate system, wherein the second tracker is associated with the anatomical object, determine, based on the second tracking data, a second pose of the second tracker (e.g., in exactly four DOF), and determine, based on the first pose and the second pose, a third pose of a virtual tracker in six DOF, the virtual tracker having a fixed spatial relationship relative to the anatomical object in the six DOF of the third pose.

According to a third aspect, a computer program product is provided. The computer program product comprises instructions which, when executed on at least one processor, cause the at least one processor to carry out the method according to the first aspect. The computer program product may be stored on one or more computer readable recording media (e.g., a hard disk, a non-transitory data storage medium or a cloud storage unit). The computer program product may be transmitted in a data stream.

According to a fourth aspect, a surgical navigation system is provided. The surgical navigation system comprises the computing system according to the third aspect.

The surgical navigation system may further comprise the first tracker and the second tracker (e.g., configured as discussed above for the first aspect). The surgical navigation system may further comprise at least one tracking system configured to provide at least one data selected from the first tracking data and the second tracking data. The at least one tracking system may comprise at least one system selected from an optical tracking system and an electromagnetic tracking system.

Each time the expression "based on" is used herein, it may have the meaning of "only based on" or "based on at least", depending on the context.

### Brief Description of the Drawings

Further details, advantages and aspects of the present disclosure will become apparent from the following embodiments taken in conjunction with the drawings, wherein:
- Fig. 1: illustrates an embodiment of a surgical navigation system in accordance with the present disclosure;
- Fig. 2A-2D: illustrate options for arranging trackers in accordance with the present disclosure;
- Fig. 3: shows an embodiment of a method in accordance with the present disclosure;
- Fig. 4: illustrates a plurality of trajectories in accordance with the present disclosure; and
- Fig. 5: illustrates a plurality of trajectories and a virtual coordinate system in accordance with the present disclosure.

### Detailed Description

In the following description, exemplary embodiments of a surgical navigation system, a method, a computing system and a computer program product will be explained with reference to the drawings. The same reference numerals will be used to denote the same or similar structural features.

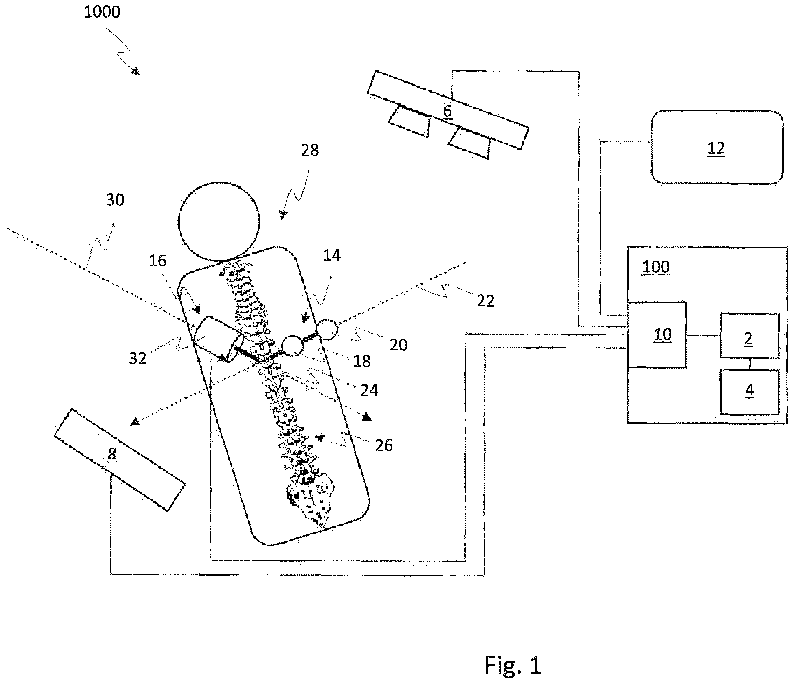

Fig. 1 illustrates an embodiment of a surgical navigation system 1000 in accordance with the present disclosure. The surgical navigation system 1000 comprises a computing system 100. The computing system 100 comprises at least one processor 2 and at least one memory 4, the at least one memory 4 storing instructions which, when executed by the at least one processor 2, configure the at least one processor 2 to perform the method(s) as described herein.

The surgical navigation system 1000 may further comprise at least one of an optical tracking system 6 and an electromagnetic tracking system 8. The optical tracking system 6 and the electromagnetic tracking system 8 may each be connected to the computing system 100 via an interface 10 of the computing system 100. As can be seen in Fig. 1, the surgical navigation system 1000 may further comprise a display 12. The display 12 may be connected to the computing system 100 via the interface 10.

The surgical navigation system 1000 may further comprise a first tracker 14 and a second tracker 16.

In the illustrated example, the first tracker 14 is an optical tracker. The first tracker 14 may comprise one, two or more passive or active optical tracking markers 18, 20 configured to be tracked by the optical tracking system 6. The optical tracking markers may 18, 20 define a line in space. For example, two or more optical tracking markers 18, 20 are arranged along the line in space. Each of the optical tracking markers 18, 20 may be configured such that only a spatial position, but no spatial orientation of the respective optical tracking marker can be determined by the optical tracking system 6.

In one example, two or more optical tracking markers 18, 20 are arranged along the line in space in predefined relative positions to one another. In another example, two or more optical tracking markers 18, 20 are arranged along the line in space in unknown relative positions to one another. In this case, one or more of the two or more optical tracking markers 18, 20 may be movable along the line in space relative to the other optical tracking markers 18, 20 of the first tracker 14. In all these examples, the line in space may be detected based on tracking a (e.g., current) position of each of the optical tracking markers of the first tracker 14 (e.g., by connecting the tracked positions or interpolating the tracked positions).

In a still further example, the first tracker 14 may comprise only one optical tracking marker 18 or 20 movably arranged along the line in space, thereby defining the line in space. In this case, the first tracker 14 may be referred to as a 3-DOF tracker. A movement of the one optical tracking marker 18 or 20 may be tracked by the optical tracking system 6 to determine the line in space. This may comprise tracking a (e.g., current) position of the one optical tracking marker 18 or 20 at (e.g., at least) two points in time, wherein the one optical tracking marker 18 or 20 is (e.g., manually or automatically) moved along the line in space between the two points in time.

In the example illustrated in Fig. 1, the second tracker 16 is an electromagnetic tracker. The second tracker 16 may comprise a coil 32. The coil 32 may be configured to be tracked by the electromagnetic tracking system 8. The coil 32 may extend or be wrapped around a central axis.

The second tracker 16 may be configured such that a spatial position and a spatial orientation in two axes can be determined by the electromagnetic tracking system 8. In this case, the second tracker 16 may be referred to as a 5-DOF tracker.

In an alternative implementation, the second tracker 16 may be configured such that only a spatial position, but no spatial orientation of the tracker 16 can be determined by the electromagnetic tracking system 8. In this case, the second tracker 16 may be referred to as a 3-DOF tracker and the coil may be movable along the central axis (e.g., even if the coil 32 does not extend around or is not wrapped around the central axis). Only tracked spatial positions of the second tracker 16, but not tracked orientations thereof, might be used in or as the second tracking data described herein. A movement of the coil 32 may be tracked by the electromagnetic tracking system 8 to determine the central axis along which the coil 32 is movably arranged. This may comprise tracking a (e.g., current) position of the coil 32 at (e.g., at least) two points in time, wherein the coil 32 is (e.g., manually or automatically) moved along the central axis between the two points in time.

In a still further implementation, the second tracker 16 may comprise two or more coils arranged along the central (e.g., tracker) axis. At least one, potentially all, of the two or more coils may have different, changeable and/or unknown orientations relative to the central axis (e.g., in this case the coils may not extend around or not be wrapped around the central axis). The central axis may extend through a center point of the two of more coils. The electromagnetic tracking system 8 may be configured to determine a spatial position of the center point of each of the two or more coils. The two or more coils of the second tracker 16 may be arranged along the central axis in predefined relative positions to one another. The two or more coils may be arranged along the central axis in unknown relative positions to one another. In this case, one or more of the two or more coils may be movable along the central axis relative to the other coils of the second tracker 16. To determine a tracked pose of the second tracker 16, only the spatial positions, but not the orientations of the two or more coils may be taken into account. In other words, only tracked spatial positions of the (e.g., center points of the) two or more coils, but not tracked orientations of the two or more coils, might be used in or as the second tracking data described herein. This may still enable determining a pose of the central axis in the tracking coordinate system of the electromagnetic tracking system 8 (e.g., by interpolating or connecting the tracked positions of the coils). In essence, this approach would be similar to the use of the two or more optical tracking markers described above, which optical tracking markers may not allow for a determination of an orientation thereof.

It is noted that both the first and second tracker 14, 16 may be optical trackers, or both the first and second tracker 14, 16 may be electromagnetic trackers. In the first case, only the optical tracking system 6 may be provided, whereas in the second case, only the electromagnetic tracking system 8 may be provided.

In one variant, the first tracker 14 may be a 5-DOF tracker, as a rotational state around the line in space defined by the optical tracking markers 18, 20 may not be determinable by the optical tracking system 6 tracking the optical tracking markers 18, 20. In another variant, the first tracker 14 may comprise at least three optical tracking markers and be configured as a 6-DOF tracker. In a still further variant, the first tracker 14 may be configured as a 3-DOF tracker, for example by comprising only a single optical tracking marker as described above. In all three variants, the first tracker 14 or at least one or more optical tracking markers 18, 20 thereof may be movably arranged along an alignment trajectory 22 having a fixed spatial relationship relative to an anatomical object 24. In this case, even if the first tracker 14 is configured as a 6-DOF tracker, a relative position between the first tracker 14 and the anatomical object 24 may be partially undefined due to the movability of the first tracker 14 or the optical tracking marker(s) thereof along the alignment trajectory 22. The line in space defined by the optical tracking marker(s) 18, 20 may coincide with or be parallel to the alignment trajectory 22. This may enable determining a (e.g., third) trajectory, as will be discussed further below.

In one variant, the second tracker 16 may be a 5-DOF tracker, as a rotational state around the central axis of the coil 32 may not be determinable by the electromagnetic tracking system 8 tracking the coil 32. In another variant, the second tracker 16 may comprise a plurality of coils and be configured as a 6-DOF tracker. In a still further variant, the second tracker 16 may be configured as a 3-DOF tracker, as described above. In all three variants, the second tracker 16 or at least one or more coils thereof may be movably arranged along an alignment trajectory 30 having a fixed spatial relationship relative to the anatomical object 24. In this case, even if the second tracker 16 is configured as a 6-DOF tracker, a relative position between the second tracker 16 and the anatomical object 24 may be partially undefined due to the movability of the second tracker 16 or the coil(s) thereof along the alignment trajectory 30. The central axis may coincide with or be parallel to the alignment trajectory 30. This may enable determining a (e.g., third) trajectory, as will be discussed further below.

In the illustrated example, the anatomical object 24 is a vertebra of a spine 26 of a human patient 28. It is noted that the anatomical object 24 does not need to be a vertebra. The anatomical object may, for example, be one of the spine 26, a bone of the patient 28, an organ of the patient 28, a limb of the patient 28, a head of the patient 28 etc. Generally speaking, the anatomical object 24 may be part of the body of the patient 28.

The first tracker 14 and the second tracker 16 do not necessarily need to be directly fixed onto the anatomical object 24. As a non-limiting example, the first tracker 14 may be movably arranged on a linear guide, the linear guide being attached to the surface of the body of the patient 28 using an adhesive patch. As another non-limiting example, the second tracker 16 may be movably arranged on a linear track attached to a surgical table, wherein the patient 28 is fixated to the surgical table (e.g., using straps). Other attachment variants of the trackers 14, 16 are also possible. That is, the first and second tracker 14, 16 may not need to be attached to the patient 28 by a substantial interaction with the body of the patient 28.

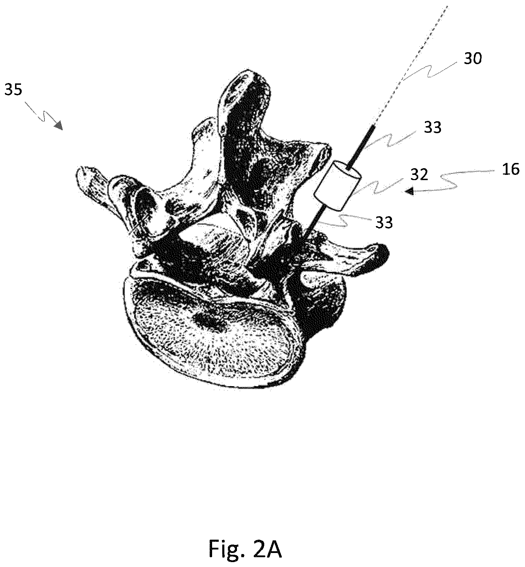

The present disclosure provides for several options for arranging the first tracker 14 and the second tracker 16 relative to the anatomical object 24. Some of these options will now be discussed with reference to Figs. 2A-2D.

As exemplarily illustrated in Fig. 2A, one or both of the first tracker 14 and the second tracker 16 may be (e.g., directly or indirectly) mechanically coupled to a linear member arranged in a fixed spatial relationship relative to the anatomical object 24, wherein the alignment trajectory 22, 30 corresponds to or is parallel to a longitudinal axis of the linear member. The linear member may, for example, be a pin, a k-wire, a nail, a drill head or a screw. The linear member may be the linear guide or the linear track mentioned above. For example, the second tracker 16 may be slid onto a pin attached to the anatomical object 24.

In the illustration of Fig. 2A, the second tracker 16 is mechanically coupled to a metal pin 33 as the linear member, the pin 33 being attached to a pedicle of a vertebra 35 of the spine 26 of the patient 28. In this example, the coil 32 is slid onto the pin 33. In the illustrated example, the alignment axis 30 coincides with the longitudinal axis of the pin. The coil 32 may be translationally movable along the pin 33. The coil 32 may be rotatable around the pin 33.

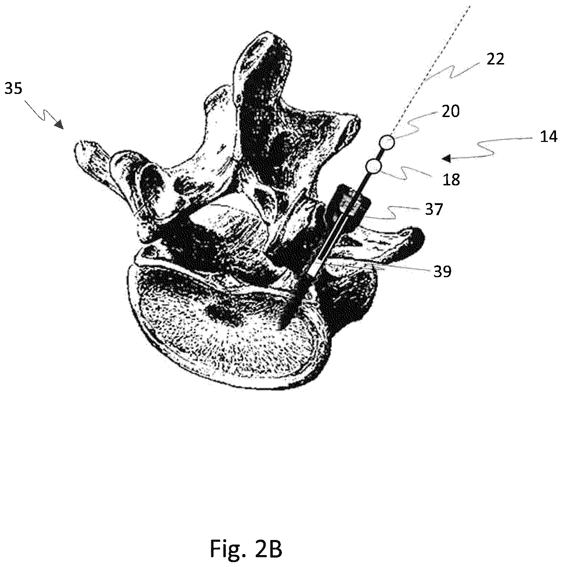

As exemplarily illustrated in Fig. 2B, one or both of the first tracker 14 and the second tracker 16 may be (e.g., directly or indirectly) mechanically coupled to a cannulated implant, the cannulated implant arranged in a fixed spatial relationship relative to the anatomical object 24, wherein the alignment trajectory 22, 30 corresponds to or is parallel to a longitudinal axis of a cannulation within the cannulated implant. The cannulated implant may, for example, be a cannulated (e.g., bone) screw, a cannulated pedicle screw, a cannulated (e.g., bone) nail, a cannulated bone replacement implant, a cannulated cage for disk replacement surgery or a cannulated prosthesis. For example, the first tracker 14 may be attached to a cannulation of a cannulated pedicle screw inserted into a vertebra of the patient's spine 26.

In the illustration of Fig. 2B, the first tracker 14 is mechanically coupled to a cannulated pedicle screw 37 inserted into vertebra 35 of the spine 26 of the patient 28. The line in space defined by the optical tracking markers 18, 20 in this example coincides with the alignment axis 22 and with the longitudinal axis of a cannulation 39 within the cannulated pedicle screw 37. The first tracker 14 may be translationally movable along the longitudinal axis of the cannulation 39. The first tracker 14 may be rotatable around the longitudinal axis of the cannulation 39.

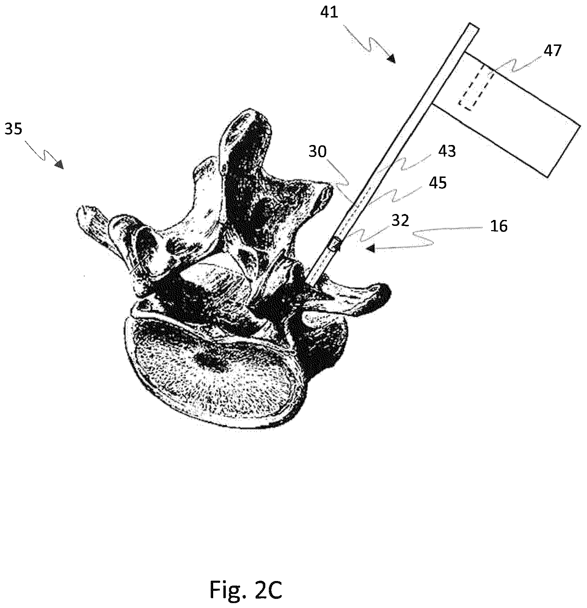

As exemplarily illustrated in Fig. 2C, one or both of the first tracker 14 and the second tracker 16 may be (e.g., directly or indirectly) mechanically coupled to a surgical instrument, the surgical instrument arranged in a fixed spatial relationship relative to the anatomical object 24, wherein the alignment trajectory 22, 30 corresponds to or is parallel to a longitudinal axis of a cannulation or tracker receptacle within the surgical instrument. The surgical instrument may be a pointer, a drill guide, a drill, a screwdriver or a guiding instrument attached to a k-wire. The longitudinal axis of the cannulation or tracker receptacle may be fixed relative to a longitudinal axis of a tip of the surgical instrument, the tip being (e.g., configured to be) in contact with the anatomical object 24 or another portion of the body of the patient 28. For example, the coil 32 of the second tracker 16 may be placed inside a cannulation extending throughout a drill guide, the drill guide being attached to the anatomical object 24.

In the illustration of Fig. 2C, the second tracker 16 is mechanically coupled to a surgical instrument 41 that is placed in a fixed spatial relationship relative to a pedicle of vertebra 35 of the spine 26 of the patient 28. In this example, the coil 32 is slid inside a cannulation 43 within an elongate shaft 45 of the surgical instrument 41. In the illustrated example, the alignment axis 30 coincides with the longitudinal axis of the cannulation 43 and the longitudinal axis of the shaft 43. The coil 32 may be translationally movable along the longitudinal axis of the cannulation 43. The coil 32 may be rotatable around the longitudinal axis of the cannulation 43.

Alternatively, the second tracker 16 may be placed inside a tracker receptacle 47 of the surgical instrument 41. The tracker receptacle 47 may have a longitudinal axis and the inserted second tracker 16 may be translationally movable along and, optionally, rotatable around the longitudinal axis of the tracker receptacle 16. In this case, the surgical instrument may not comprise the cannulation 43.

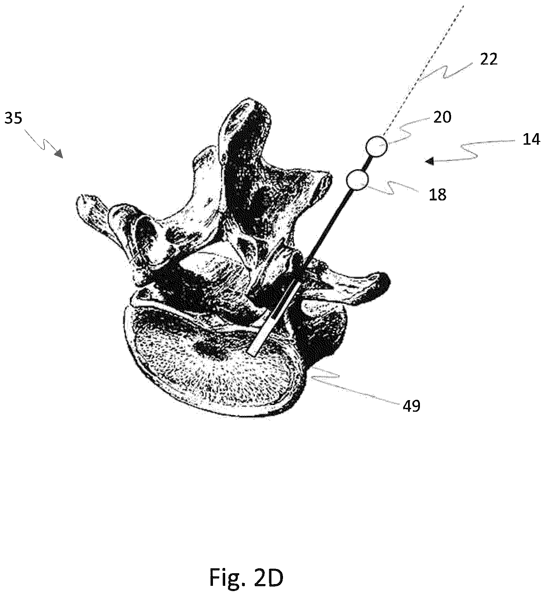

As exemplarily illustrated in Fig. 2D, one or both of the first tracker 14 and the second tracker 16 may be (e.g., directly or indirectly) mechanically coupled to a linear hole in the anatomical object 24, wherein the alignment trajectory 22, 30 corresponds to or is parallel to a longitudinal axis of the linear hole. The linear hole may be cylindrical. The linear hole may, for example, be a drilled hole or a hole naturally occurring within the anatomical object 24. For example, the second tracker 16 may be placed inside a hole drilled into a pedicle of a vertebra of the patient's spine 26.

In the illustration of Fig. 2D, the first tracker 14 is mechanically coupled to a linear hole 49 drilled into vertebra 35 of the spine 26 of the patient 28. The line in space defined by the optical tracking markers 18, 20 in this example coincides with the alignment axis 22 and with the longitudinal axis of the hole 49. The first tracker 14 may be translationally movable along the longitudinal axis of the hole 49. The first tracker 14 may be rotatable around the longitudinal axis of the hole 49.

It is noted that one or both of the trackers 14, 16 may be arranged according to two or more of the above options. For example, the first tracker 14 may be mechanically coupled to both the linear member and the cannulated implant. As another example, the second tracker 16 may be mechanically coupled to both the surgical instrument and the linear hole.

Using the above trackers 14, 16 may be advantageous, as such trackers may not need to be arranged in a fixed position relative to the anatomical object 24, but merely along the respective alignment trajectories 22, 30. For example, the first tracker 14 may be arranged in an arbitrary axial position along the alignment trajectory 22 by coupling the first tracker 14 onto the cannulation 39 within the cannulated pedicle screw 37 inserted in the vertebra 35. As another example, the second tracker 16 may be arranged in an arbitrary axial position along the alignment trajectory 30 by sliding the coil 32 onto the metal pin 33 attached to the vertebra of the spine 26.

As apparent from the above, each of the first tracker 14 and the second tracker 16 individually may not enable tracking a pose of the anatomical object 24 in 6 DOF. For instance, the trackers 14, 16 may be 3-DOF or 5-DOF trackers. Alternatively or additionally, the trackers 14, 16 (or, e.g., the coil(s) or optical tracking marker(s) thereof) may be translationally and/or rotationally movable in relation to the respective alignment trajectory 22, 30 that has a fixed spatial relationship to the anatomical object 24. Coils of the trackers 16 may have changeable or unknown orientations relative to the respective alignment trajectory 30. Even if the trackers as such are configured as 6-DOF trackers, the movability of the trackers may prohibit determining a relative pose between the anatomical object 24 and a tracker in 6 DOF when using just one of the trackers.

The present disclosure provides for an advantageous method which combines tracking data of two (e.g., 3-, 5- or 6-DOF) trackers for determining a single virtual tracker. In particular, the method described herein may allow using the above first and second tracker 14, 16 for determining a pose of a virtual 6-DOF tracker that has a fixed pose in 6 DOF relative to the anatomical object 24. The virtual 6-DOF tracker may be used as a reference during surgical navigation, for example for displaying representations of tracked surgical instruments relative to the virtual 6-DOF tracker. The virtual 6-DOF tracker may also be registered to image data of the anatomical object 24, which means that a transformation between the virtual 6-DOF tracker and a coordinate system of the image data may be determined or obtained. The method may thus allow determining a pose of the coordinate system of the image data relative to the tracking system(s) used, even if the individual trackers are translationally and/or rotationally movable in relation to the respective alignment trajectory 22, 30.

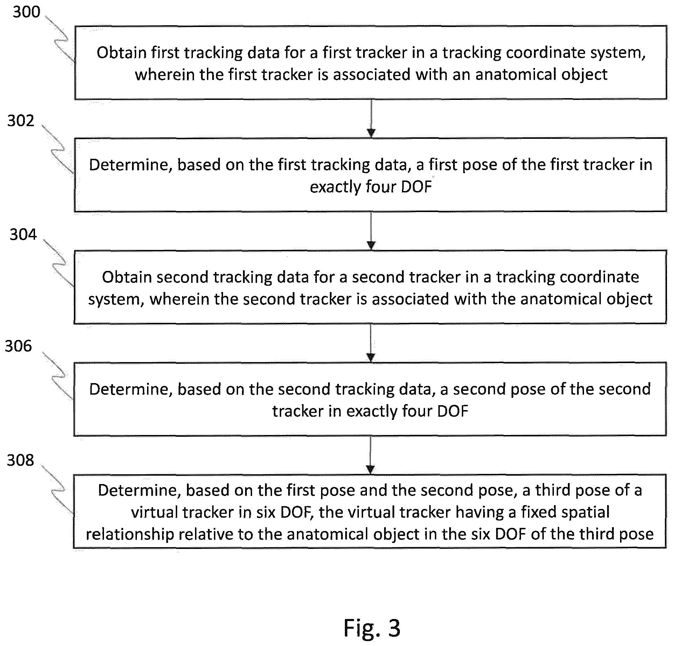

Fig. 3 shows an embodiment of a method in accordance with the present disclosure. The method may be performed by the at least one processor 2 of the computing system 100, or by another processor.

In step 300, first tracking data for the first tracker 14 in a tracking coordinate system is obtained. The first tracker 14 is associated with the anatomical object 24, as explained above. The first tracking data may be obtained from the optical tracking system 6. The tracking coordinate system may be associated with the optical tracking system 6 or may be a global tracking coordinate system associated with a plurality of tracking systems (e.g., both tracking systems 6 and 8). A first known transformation may be used to transform a pose (e.g., a tracked pose of the first tracker 14) from the tracking coordinate system of the optical tracking system 6 to the global tracking coordinate system and vice versa.

In step 302, based on the first tracking data, a first pose of the first tracker 14 is determined in exactly four DOF. The determined first pose may define a fixed spatial relationship of the first tracker 14 relative to the anatomical object 24 in only the four DOF of the first pose. The four DOF of the first pose must comprise two translational DOF and two rotational DOF.

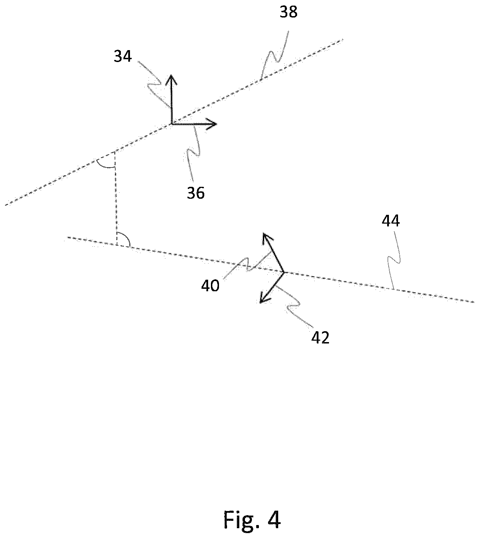

As schematically illustrated in Fig. 4, a first of the two translational DOF of the first pose and a first of the two rotational DOF of the first pose may be associated with a first trajectory 34. This means that the first of the two translational DOF of the first pose may correspond to a translation along the first trajectory 34, and the first of the two rotational DOF of the first pose may correspond to a rotation around the first trajectory 34.

A second of the two translational DOF of the first pose and a second of the two rotational DOF of the first pose may be associated with a second trajectory 36. The second trajectory 36 may orthogonal to the first trajectory 34. Similarly, this means that the second of the two translational DOF of the first pose may correspond to a translation along the second trajectory 36, and the second of the two rotational DOF of the first pose may correspond to a rotation around the second trajectory 36.

One may say that the first pose comprises a defined position along the first trajectory 34, a defined position along the second trajectory 36, a defined rotational around the first trajectory 34 and a defined rotation around the second trajectory 36.

The only two remaining DOF that are undefined by the first pose may correspond to a translation along a third trajectory 38 and a rotation around the third trajectory 38. The third trajectory 38 may be orthogonal to both the first trajectory 34 and the second trajectory 36. The alignment trajectory 22 may coincide with the third trajectory 38.

In step 304, second tracking data for the second tracker 16 in a tracking coordinate system is obtained. The second tracker 16 is associated with the anatomical object 24, as explained above. The second tracking data may be obtained from the electromagnetic tracking system 8. The tracking coordinate system may be associated with the electromagnetic tracking system 8 or may be the global tracking coordinate system. A second known transformation may be used to transform a pose (e.g., a tracked pose of the second tracker 16) from the tracking coordinate system of the electromagnetic tracking system 8 to the global tracking coordinate system and vice versa.

The first tracking data and the second tracking data may be tracking data for the respective tracker 14, 16 in a same (e.g., the global) tracking coordinate system. The step 300 may comprise obtaining a tracked pose of the first tracker 14 in the tracking coordinate system of the optical tracking system 6, and transforming the tracked pose into the global tracking coordinate system using the first known transformation or into the tracking coordinate system of the electromagnetic tracking system 8 using a third known transformation, to determine the first tracking data. The step 304 may comprise obtaining a tracked pose of the second tracker 16 in the tracking coordinate system of the electromagnetic tracking system 6, and transforming the tracked pose into the global tracking coordinate system using the second known transformation or into the tracking coordinate system of the optical tracking system 6 using a fourth known transformation, to determine the second tracking data. The fourth known transformation may be an inverse of the third known transformation.

Different techniques of how to determine the first, second, third and/or fourth known transformation fall within the scope of the present disclosure. Four exemplary techniques will now be explained in more detail, although various other techniques may be used.

In a first technique, optical tracking markers (not shown) may be attached to the electromagnetic tracking system 8 in known locations relative to the tracking coordinate system of the electromagnetic tracking system 8. A pose of the tracking coordinate system of the electromagnetic tracking system 8 may then be determined based on tracked positions of these attached optical tracking markers in the tracking coordinate system of the optical tracking system 6, and based on their known locations relative to the tracking coordinate system of the electromagnetic tracking system 8. The pose of the tracking coordinate system of the electromagnetic tracking system 8 in the tracking coordinate system of the optical tracking system 6 may then be used as or for determining the first, second, third and/or fourth known transformation.

In a second technique, one or more electromagnetic trackers (not shown) may be attached to the optical tracking system 6 in known locations relative to the tracking coordinate system of the optical tracking system 6. A pose of the tracking coordinate system of the optical tracking system 6 may then be determined based on tracked positions of these attached trackers in the tracking coordinate system of the electromagnetic tracking system 8, and based on their known locations relative to the tracking coordinate system of the optical tracking system 6. The pose of the tracking coordinate system of the optical tracking system 8 in the tracking coordinate system of the electromagnetic tracking system 8 may then be used as or for determining the first, second, third and/or fourth known transformation.

In a third technique, the optical tracking system 6 is arranged in a fixed spatial pose relative to the electromagnetic tracking system 8 such that the tracking coordinate system of the optical tracking system 6 is arranged in a predefined spatial pose relative to the tracking coordinate system of the electromagnetic tracking system 8. The predefined spatial pose may then be used as or for determining the first, second, third and/or fourth known transformation.

In a fourth technique, a hybrid registration device is provided. The hybrid registration device is configured to be tracked by both the optical tracking system 6 and the electromagnetic tracking system 8. For example, the hybrid registration device comprises one or more optical tracking markers and one or more electromagnetic trackers, each arranged in predefined fixed positions on or in the hybrid registration device. By tracking poses of the one or more optical tracking markers via the optical tracking system 6, and based on the known predefined fixed positions thereof relative to the hybrid registration device, a pose of the hybrid registration device in the tracking coordinate system of the optical tracking system 6 may be determined. Similarly, by tracking poses of the one or more electromagnetic trackers via the electromagnetic tracking system 8, and based on the known predefined fixed positions thereof relative to the hybrid registration device, a pose of the hybrid registration device in the tracking coordinate system of the electromagnetic tracking system 8 may be determined. Based on the determined poses of the hybrid registration device in the tracking coordinate system of the optical tracking system 6 and in the tracking coordinate system of the electromagnetic tracking system 8, the first, second, third and/or fourth known transformation may be determined. The hybrid registration device may define (e.g., be fixed relative to) the global coordinate system.

Again, it is noted that instead of an optical tracker 14 and an electromagnetic tracker 16, both trackers may use the same tracking modality. In other words, both trackers 14, 16 may be optical trackers (e.g., configured similar to the optical tracker 14 described herein) or both trackers 14, 16 may be electromagnetic trackers (e.g., configured similar to the electromagnetic tracker 16 described herein). Depending on the trackers used, the first and second tracking data may be obtained from a same or from different tracking systems.

In step 306, based on the second tracking data, a second pose of the second tracker 16 is determined in exactly four DOF. The determined second pose may define a fixed spatial relationship of the second tracker 16 relative to the anatomical object 24 in only the four DOF of the second pose. The four DOF of the second pose may comprise two translational DOF and two rotational DOF.

The first pose of the first tracker 14 and the second pose of the second tracker 16 may be determined in a same coordinate system, for example in the global tracking coordinate system, in the tracking coordinate system of the optical tracking system 6 or in the tracking coordinate system of the electromagnetic tracking system 8. A pose of the first tracker 14 may be determined (e.g., in the coordinate system the optical tracking system 6) and then be transformed into the same coordinate system using at least one of the first and the third known transformation, thereby determining the first pose. A pose of the second tracker 16 may be determined (e.g., in the coordinate system the electromagnetic tracking system 8) and then be transformed into the same coordinate system using at least one of the second and the fourth known transformation, thereby determining the second pose.

Again referring to Fig. 4, a first of the two translational DOF of the second pose and a first of the two rotational DOF of the second pose may be associated with a first trajectory 40. This means that the first of the two translational DOF of the second pose may correspond to a translation along the first trajectory 40, and the first of the two rotational DOF of the second pose may correspond to a rotation around the first trajectory 40.

A second of the two translational DOF of the second pose and a second of the two rotational DOF of the second pose may be associated with a second trajectory 42. The second trajectory 42 may be orthogonal to the first trajectory 40. Similarly, this means that the second of the two translational DOF of the second pose may correspond to a translation along the second trajectory 42, and the second of the two rotational DOF of the second pose may correspond to a rotation around the second trajectory 42.

One may say that the second pose comprises a defined position along the first trajectory 40, a defined position along the second trajectory 42, a defined rotation around the first trajectory 40 and a defined rotation around the second trajectory 42.

The only two remaining DOF that are undefined by the second pose may correspond to a translation along a third trajectory 44 and a rotation around the third trajectory 44. The third trajectory 44 may be orthogonal to both the first trajectory 40 and the second trajectory 42. The alignment trajectory 30 may coincide with the third trajectory 44.

In step 308, based on the first pose and the second pose, a third pose of a virtual tracker is determined in six DOF, the virtual tracker having a fixed spatial relationship relative to the anatomical object 24 in the six DOF of the third pose. The virtual tracker may be referred to as a (e.g., virtual) 6-DOF tracker.

The order of steps 300-306 may be changed, but step 300 shall be performed before step 302, and step 304 shall be performed before step 306.

Step 308 may comprise determining, based on the first pose, the third trajectory 38 defined by the first tracker 14 and associated with the first pose. The first trajectory 34 may be orthogonal to the second trajectory 36, and the third trajectory 38 may be orthogonal to the first trajectory 34 and the second trajectory 36, as illustrated in Fig. 3. As noted above, the only two remaining DOF that are undefined by the first pose may correspond to a translation along the third trajectory 38 and a rotation around the third trajectory 38. In other words, the third trajectory 38 may be associated with at least one of a translational and a rotational DOF excluded by the exactly four DOF of the first pose of the first tracker 14. The alignment axis 22 may be parallel to or coincide with the third trajectory 38. The alignment axis 22 may be associated with a translational and a rotational DOF excluded by the exactly four DOF of the first pose of the first tracker 14.

Step 308 may further comprise determining, based on the second pose, the third trajectory 44 defined by the second tracker 16 and associated with the second pose. The first trajectory 40 may be orthogonal to the second trajectory 42, and the third trajectory 44 may be orthogonal to the first trajectory 40 and the second trajectory 42, as illustrated in Fig. 3. As noted above, the only two remaining DOF that are undefined by the second pose may correspond to a translation along the third trajectory 44 and a rotation around the third trajectory 44. In other words, the third trajectory 44 may be associated with at least one of a translational and a rotational DOF excluded by the exactly four DOF of the second pose of the second tracker 16. The alignment axis 30 may be parallel to or coincide with the third trajectory 44. The alignment axis 30 may be associated with a translational and a rotational DOF excluded by the exactly four DOF of the second pose of the second tracker 16.

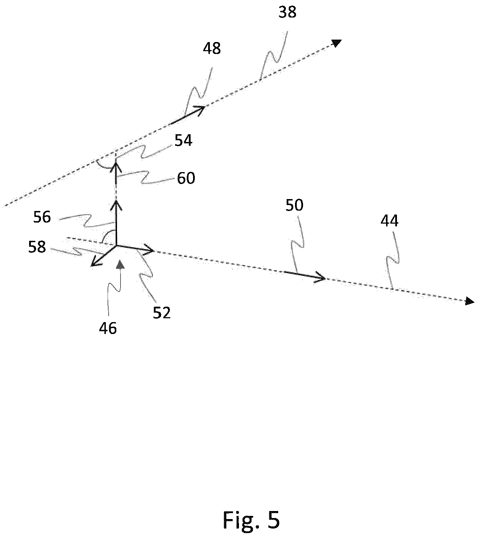

The first and second tracker 14, 16 may be arranged movably along alignment axes 22, 30 that are non-parallel (e.g., skew) relative to one another. As can be seen in Fig. 4, the two third trajectories 38, 44 may be skew relative to one another. This may be advantageous for determining the third pose of the virtual tracker in six DOF. Details of how the third pose may be determined based on the first pose and the second pose will now be explained with reference to Fig. 5.

Fig. 5 shows a virtual coordinate system 46 having a predefined spatial relationship relative to the third pose of the virtual tracker. One may say that the virtual coordinate system 46 corresponds to, defines or is indicative of the third pose of the virtual tracker.

The third trajectories 38, 44 may be used to determine the virtual coordinate system 46 in the tracking coordinate system. As noted above, the tracking coordinate system may be a coordinate system associated with the optical tracking system 6, a coordinate system associated with the electromagnetic tracking system 8 or a (e.g., global) coordinate system associated with both the optical tracking system 6 and the electromagnetic tracking system 8. Determining the virtual coordinate system 46 may involve determining a first directional vector 48 of the third trajectory 38 and a second directional vector 50 of the third trajectory 44. Which of the two possible directions along the respective third trajectories 38, 44 to use for determining the directional vectors 48, 50 may be arbitrarily chosen. That is, the directional vectors 48, 50 may extend in the opposite direction compared with Fig. 5.

A first coordinate axis of the virtual coordinate system 46 may be defined to coincide with one of the third trajectory 38 and the third trajectory 44. In the example shown in Fig. 5, the first coordinate axis 52 of the virtual coordinate system 46 coincides with the third trajectory 44.

Determining the virtual coordinate system 46 may comprise determining a direction 54 of a smallest distance between the third trajectory 38 and the third trajectory 44, and defining a second coordinate axis 56 of the virtual coordinate system 46 to coincide with the direction 54. If the third trajectories 38, 44 intersect, the second coordinate axis 56 may be defined to coincide with a normal of a plane in which both trajectories 38, 44 lie.

Determining the virtual coordinate system 46 may comprise defining a third coordinate axis 58 of the virtual coordinate system 46 such that it corresponds to a cross product between the second directional vector 50 and a third directional vector 60 extending along the direction 54 or the normal. Alternatively, the third coordinate system 58 may be defined such that it corresponds to a cross product between the third directional vector 60 and the second directional vector 50. The third directional vector 60 may extend, relative to the third trajectory 44 coinciding with the first coordinate axis 52, into the direction of the third trajectory 38, or into the opposite direction.

One may say that the virtual coordinate system 46 defines the third pose in six DOF relative to (e.g., the combination of) the third trajectory 38 and the third trajectory 44. The axial position of the trackers 14, 16 along the respective alignment axes 22, 30, which axes 22, 30 may be parallel to or may coincide with the third trajectories 38, 44, thus may have no influence on the third pose defined by the virtual coordinate system 46. In other words, the method described herein may allow determining the third pose of the virtual tracker (e.g., defined by the virtual coordinate system 46) in six DOF, the third pose having a fixed spatial relationship relative to the anatomical object 24, despite a movability of the trackers 14, 16 relative to the anatomical object 24.

One or both of the trackers 14, 16 may be 5-DOF trackers or 6-DOF trackers, but one or more DOF defined by the trackers 14, 16 may be disregarded due to the tracker's movability relative to the anatomical object 24. The first tracking data may be indicative of a tracked pose of the first tracker 14 in five or six DOF, and determining the first pose may comprise selectively disregarding one or two of the DOF of the tracked pose of the first tracker 14 to obtain the first pose in the exactly four DOF. Alternatively or additionally, the second tracking data may be indicative of a tracked pose of the second tracker 16 in five or six DOF, and determining the second pose may comprise selectively disregarding one or two of the DOF of the tracked pose of the second tracker 16 to obtain the second pose in the exactly four DOF. The method may comprise obtaining tracker information data defining which of the one or two DOF to disregard.

As described above, one or both of the trackers 14, 16 may be 3-DOF trackers. A first temporary pose and a second temporary pose of the (e.g., first or second) tracker 14, 16 may be obtained. The first temporary pose may define a spatial position of the tracker 14, 16 in three DOF in the tracking coordinate system at a first point in time. The second temporary pose may define a spatial position of the tracker 14, 16 in three DOF in the tracking coordinate system at a later second point in time. The first temporary pose may differ from the second temporary pose. Based on the first temporary pose and the second temporary pose, the (e.g., first or second) tracking data may be determined. The tracking data may, as described above, be indicative of a tracked pose of the tracker 14, 16 in five or six DOF. Based on the so-determined tracking data, or based on the first temporary pose and the second temporary pose, the (e.g., first or second) pose of the (e.g., first or second) tracker 14, 16 may be determined in the exactly four DOF (e.g., in step 302 or 306). This may involve selectively disregarding one or two of the DOFs of the tracked pose indicated by the tracking data, as described above.

Put in other words, a movement of the tracker 14, 16 between the first point in time and the second point in time may define a movement axis. The movement axis may then be used similar to the line in space of the 5-DOF tracker comprising two optical tracking markers 18, 20. For example, the only DOFs excluded by the (e.g., first or second) pose of the (e.g., first or second) tracker 14, 16 may correspond to a translation along and a rotation around the movement axis. The movement axis may correspond to the line in space defined by the optical tracking marker(s) or the central axis of the coil 32.

In other words, one may use a 3-DOF tracker comprising only one optical tracking marker 18 or 20 and use different positions of the 3-DOF tracker, tracked at different points in time, to determine a tracked pose of the tracker in more than 3 DOF (e.g., 4 DOF or 5 DOF). The first tracking data may be indicative of the so-determined tracked pose of the first tracker 14 in 5 DOF. One of the 5 DOFs may be selectively disregarded to determine the first pose of the first tracker 14 in the exactly 4 DOF, as explained above. The same may be applicable for the second tracking data and the second pose of the second tracker 16.

A similar technique may be used in combination with a 5-DOF or a 6-DOF tracker. That is, even in case of such a 5-DOF or 6-DOF tracker, the movement of the tracker along the respective movement axis may be tracked. In particular, a first temporary pose and a second temporary pose of the (e.g., first or second) tracker 14, 16 may be obtained. The first temporary pose may define a spatial position of the tracker 14, 16 in five or six DOF in the tracking coordinate system at a first point in time. The second temporary pose may define a spatial position of the tracker 14, 16 in five or six DOF in the tracking coordinate system at a later second point in time. The first temporary pose may differ from the second temporary pose. Based on the first temporary pose and the second temporary pose, the (e.g., first or second) tracking data may be determined.

For example, the tracker information of the (e.g., first or second) tracker 14, 16 may define that a particular translational DOF associated with a tracker axis and a particular rotational DOF associated with the tracker axis are to be disregarded. In this case, the tracker 14, 16 may be movably arranged with respect to the tracker axis. The tracker axis may correspond to at least one of the line in space, the central axis, the movement axis and the alignment axis described herein. The tracker axis of a particular tracker 14, 16 may coincide with the third trajectory determined based on the tracking data of the particular tracker 14, 16. In case the movement of the (e.g., 3-DOF, 5-DOF or 6-DOF) tracker is tracked as noted above, no tracker information may need to be obtained. Instead, the movement axis, derived from the tracking data (e.g., that is determined based on the first temporary pose and the second temporary pose), may be considered as defining a translational DOF and a rotational DOF that are excluded by the first or second pose of the tracker determined in the exactly 4 DOF.

The method may further comprise determining or obtaining a transformation between (i) a fourth pose of the anatomical object 24 in six DOF in an image coordinate system of image data of the anatomical object 24 and (ii) the third pose in the tracking coordinate system.

Obtaining the transformation may comprise transferring an existing registration from a 6-DOF registration tracker to the virtual tracker. In this case, a sixth pose of the registration tracker may be obtained in six DOF in the tracking coordinate system, the registration tracker having a fixed spatial relationship relative to the anatomical object 24. An existing transformation between the fourth pose and the sixth pose may be obtained. One or more intermediate transformation(s) between the sixth pose and at least one of the first pose and the second pose may be determined. Next, the transformation between (i) and (ii) may be determined based on the existing transformation and the intermediate transformation(s).

Determining the transformation may comprise detecting the alignment trajectories 22 and 30 in the image data. As described above, each alignment trajectory may have a known spatial relationship relative to the respective tracker 14, 16, as the trackers 14, 16 may each be movably arranged along one of the alignment trajectories 22, 30. The detected alignment trajectory 22 may be matched to the pose of the first tracker 14 and the detected alignment trajectory 30 may be matched to the pose of the second tracker 16 using the known spatial relationship, thereby determining the transformation. The known spatial relationship may define that each alignment trajectory 22, 30 coincides with the respective third trajectory 38, 44. In this case, the detected alignment trajectories 22, 30 may be matched to the respective third trajectory 38, 44 to determine the transformation.

The alignment trajectories 22, 30 may be detected in the image by conducting an image analysis to identify at least one component chosen from the linear member, the cannulated implant, the surgical instrument arranged in a fixed spatial relationship relative to the anatomical object 24, and the a linear hole in the anatomical object 24. A predefined spatial relationship between the identified component and the alignment axis 22 or 30 may be used to detect the alignment axis 22, 30 in the image data. For example, the pin may be detected in the image data and a longitudinal axis thereof may set as the alignment axis 30 in case the coil 32 of the second tracker 16 is slid onto the pin (e.g., after having acquired the image data). In this manner, a registration between the virtual tracker and the image data may be obtained even without attaching a tracker on the anatomical element 24 during image acquisition.

The method may further comprise determining a fifth pose of the anatomical object in six DOF in the tracking coordinate system based on the third pose and the transformation. This may enable tracking the anatomical object 24 in the tracking coordinate system. Furthermore, this may enable tracking a surgical instrument relative to the anatomical object 24 as described by the image data. Thus, a pose of the tracked surgical instrument may be determined relative to the image data and an indication thereof may be displayed on the display 12.

As apparent from the above, the present disclosure provides for an advantageous method for use in surgical navigation. The method may combine tracked poses of two or more 3-DOF trackers, 4-DOF trackers, 5-DOF trackers or 6-DOF trackers into a third pose of a virtual tracker having a fixed relationship relative to an anatomical object in six DOF. The method may allow use of 3-DOF trackers, 4-DOF trackers, 5-DOF trackers or 6-DOF trackers that are movably arranged with respect to an alignment axis having a fixed spatial pose relative to the anatomical object. This may allow using compact low-cost trackers. The method may not require an accurate and stable positioning of the trackers along the alignment axis. Furthermore, the alignment axes may be defined by existing (e.g., drill) holes or components (e.g., inserted cannulated pedicle screws or attached pins). This may improve usability for a surgeon and ensure accurate tracking results. A registration between image data of an anatomical element and the virtual tracker may be determined without attaching trackers to the anatomical element during image acquisition and without requiring a registration point acquisition on the patient's body by a surgeon. The present disclosure is not limited to these advantageous effects. Additional advantages may become apparent to the skilled person when referring to the present disclosure.

## Claims

1. A method for use in surgical navigation, the method being performed by a computing system (100) and comprising:
obtaining (300) first tracking data for a first tracker (14) in a tracking coordinate system, wherein the first tracker (14) is associated with an anatomical object (24);
determining (302), based on the first tracking data, a first pose of the first tracker (14) in exactly four degrees of freedom, DOF, wherein the four DOF of the first pose comprise two translational DOF and two rotational DOF;
obtaining (304) second tracking data for a second tracker (16) in a tracking coordinate system, wherein the second tracker (16) is associated with the anatomical object (24);
determining (306), based on the second tracking data, a second pose of the second tracker in exactly four DOF, wherein the four DOF of the second pose comprise two translational DOF and two rotational DOF;
determining (308), based on the first pose and the second pose, a third pose of a virtual tracker in six DOF, the virtual tracker having a fixed spatial relationship relative to the anatomical object (24) in the six DOF of the third pose.

2. The method of claim 1, further comprising:
determining or obtaining a transformation between (i) a fourth pose of the anatomical object (24) in six DOF in an image coordinate system of image data of the anatomical object (24) and (ii) the third pose.

3. The method of claim 2, further comprising:
determining a fifth pose of the anatomical object (24) in six DOF based on the third pose and the transformation.

4. The method of any one of claims 1 to 3, wherein
the determined first pose defines a fixed spatial relationship of the first tracker (14) relative to the anatomical object (24) in only the four DOF of the first pose, and wherein the determined second pose defines a fixed spatial relationship of the second tracker (16) relative to the anatomical object (24) in only the four DOF of the second pose.

5. The method of any one of claims 1 to 4, wherein
a first of the two translational DOF and a first of the two rotational DOF are associated with a first trajectory (34; 40), and wherein a second of the two translational DOF and a second of the two rotational DOF are associated with a second trajectory (36; 42) different from the first trajectory.

6. The method of claim 5, wherein
determining the third pose of the virtual tracker comprises:
determining, based on one of the first pose and the second pose, a third trajectory (38; 44) defined by the first or second tracker (14; 16) associated with the one of the first pose and the second pose, the third trajectory (38; 44) being different from the first trajectory (34; 40) and the second trajectory (36; 42); and
determining the third pose of the virtual tracker further based on the third trajectory (38; 44).

7. The method of claim 6, wherein
determining the third pose of the virtual tracker comprises:
determining, based on the first pose, a first instance of the third trajectory (34);
determining, based on the second pose, a second instance of the third trajectory (44), wherein the second instance of the third trajectory (44) is non-parallel relative to the first instance of the third trajectory (34); and
determining, based on the first instance of the third trajectory (34) and the second instance of the third trajectory (38), a virtual coordinate system (46), the virtual coordinate system (46) having a predefined spatial relationship relative to the third pose of the virtual tracker.

8. The method of claim 7, wherein
determining the virtual coordinate system comprises determining a first directional vector (48) of the first instance of the third trajectory (38), determining a second directional vector (50) of the second instance of the third trajectory (44), and defining a coordinate axis (52; 58) of the virtual coordinate system (46) to lie within a plane spanned by the first directional vector (48) and the second directional vector (50).

9. The method of claim 7 or 8, wherein
determining the virtual coordinate system (46) comprises defining a coordinate axis (52) of the virtual coordinate system (46) to coincide with one of the first instance of the third trajectory (38) and the second instance of the third trajectory (44).

10. The method of any one of claims 7 to 9, wherein
determining the virtual coordinate system (46) comprises determining a direction (54) of a smallest distance between the first instance of the third trajectory (38) and the second instance of the third trajectory (44), and defining a coordinate axis (56) of the virtual coordinate system (46) to coincide with the direction (56).

11. The method of claim 2 or any one of claims 3 to 10 as far as depending on claim 2, wherein
determining the transformation comprises:
detecting an alignment trajectory (22; 30) in the image data, the alignment trajectory (22; 30) having a known spatial relationship relative to at least one of the first tracker (14) and the second tracker (16); and
matching the detected alignment trajectory (22; 30) to the pose of the at least one of the first tracker (14) and the second tracker (16), using the known spatial relationship.

12. The method of any one of claims 1 to 11, wherein
at least one of the first tracker (14) and the second tracker (16) is movably arranged along an alignment trajectory (22; 30) having a fixed spatial relationship relative to the anatomical object (24).

13. The method of claim 11 or 12, wherein
the at least one of the first tracker (14) and the second tracker (16) is mechanically coupled to one or more elements chosen from
(i) a linear member (33) arranged in a fixed spatial relationship relative to the anatomical object (24), wherein the alignment trajectory (22; 30) corresponds to or is parallel to a longitudinal axis of the linear member (33);
(ii) a cannulated implant (37), the cannulated implant (37) arranged in a fixed spatial relationship relative to the anatomical object (24), wherein the alignment trajectory (22; 30) corresponds to or is parallel to a longitudinal axis of a cannulation (39) within the cannulated implant (37);
(iii) a surgical instrument (41), the surgical instrument (41) arranged in a fixed spatial relationship relative to the anatomical object (24), wherein the alignment trajectory (22; 30) corresponds to or is parallel to a longitudinal axis of a cannulation (43) or tracker receptacle (47) within the surgical instrument (41); and
(iv) a linear hole (49) in the anatomical object (24), wherein the alignment trajectory (22; 30) corresponds to or is parallel to a longitudinal axis of the linear hole (49).

14. The method of any one of claims 1 to 13, wherein
the first tracking data is indicative of a tracked pose of the first tracker (14) in five or six DOF, and wherein determining the first pose comprises selectively disregarding one or two of the DOF of the tracked pose of the first tracker (14) to obtain the first pose in the exactly four DOF; and/or
wherein the second tracking data is indicative of a tracked pose of the second tracker (16) in five or six DOF, and wherein determining the second pose comprises selectively disregarding one or two of the DOF of the tracked pose of the second tracker (16) to obtain the second pose in the exactly four DOF.

15. The method of any one of claims 1 to 14, wherein
at least one of the first tracker (14) and the second tracker (16) is a 3-DOF, 4-DOF or 5-DOF tracker comprising a coil (32), wherein the coil (32) is configured to be tracked by an electromagnetic tracking system (8); and/or
at least one of the first tracker (14) and the second tracker (16) is a 3-DOF, 4-DOF or 5-DOF tracker comprising one, two or more optical tracking markers (18, 20) defining a line in space, wherein the markers (18, 20) are configured to be tracked by an optical tracking system (6).

16. The method of any one of claims 1 to 15, wherein
the anatomical object (24) comprises or is a vertebra of a spine (26) of a human patient (28).

17. A computing system (100) comprising at least one processor (2) and at least one memory (4), the at least one memory (4) storing instructions which, when executed by the at least one processor (2), configure the at least one processor (2) to:
obtain (300) first tracking data for a first tracker (14) in a tracking coordinate system, wherein the first tracker (14) is associated with an anatomical object (24);
determine (302), based on the first tracking data, a first pose of the first tracker (14) in exactly four degrees of freedom, DOF, wherein the four DOF of the first pose comprise two translational DOF and two rotational DOF;
obtain (304) second tracking data for a second tracker (16) in a tracking coordinate system, wherein the second tracker (16) is associated with the anatomical object (24);
determine (306), based on the second tracking data, a second pose of the second tracker (16) in exactly four DOF, wherein the four DOF of the second pose comprise two translational DOF and two rotational DOF;
determine (308), based on the first pose and the second pose, a third pose of a virtual tracker in six DOF, the virtual tracker having a fixed spatial relationship relative to the anatomical object (24) in the six DOF of the third pose.

18. The computing system (100) of claim 17, further configured to perform the method according to any one of claims 2 to 16.

19. A computer program product comprising instructions which, when executed on at least one processor (2), cause the at least one processor (2) to carry out the method according to any one of claims 1 to 16.

20. The computer program product of claim 19, stored on one or more computer readable recording media (4).

## Patentansprüche

1. Verfahren zur Verwendung in der chirurgischen Navigation, wobei das Verfahren durch ein Computersystem (100) durchgeführt wird und umfasst:
Erlangen (300) von ersten Tracking-Daten für einen ersten Tracker (14) in einem Tracking-Koordinatensystem, wobei der erste Tracker (14) mit einem anatomischen Objekt (24) assoziiert ist;
Bestimmen (302), basierend auf den ersten Tracking-Daten, einer ersten Pose des ersten Trackers (14) in genau vier Freiheitsgraden, DOF, wobei die vier DOF der ersten Pose zwei translatorische DOF und zwei rotatorische DOF umfassen;
Erlangen (304) von zweiten Tracking-Daten für einen zweiten Tracker (16) in einem Tracking-Koordinatensystem, wobei der zweite Tracker (16) mit dem anatomischen Objekt (24) assoziiert ist;
Bestimmen (306), basierend auf den zweiten Tracking-Daten, einer zweiten Pose des zweiten Trackers in genau vier DOF, wobei die vier DOF der zweiten Pose zwei translatorische DOF und zwei rotatorische DOF umfassen;
Bestimmen (308), basierend auf der ersten Pose und der zweiten Pose, einer dritten Pose eines virtuellen Trackers in sechs DOF, wobei der virtuelle Tracker eine feste räumliche Beziehung relativ zu dem anatomischen Objekt (24) in den sechs DOF der dritten Pose aufweist.

2. Verfahren nach Anspruch 1, ferner umfassend:
Bestimmen oder Erlangen einer Transformation zwischen (i) einer vierten Pose des anatomischen Objekts (24) in sechs DOF in einem Bildkoordinatensystem von Bilddaten des anatomischen Objekts (24) und (ii) der dritten Pose.

3. Verfahren nach Anspruch 2, ferner umfassend:
Bestimmen einer fünften Pose des anatomischen Objekts (24) in sechs DOF auf der Grundlage der dritten Pose und der Transformation.

4. Verfahren nach einem der Ansprüche 1 bis 3, wobei
die bestimmte erste Pose eine feste räumliche Beziehung des ersten Trackers (14) relativ zu dem anatomischen Objekt (24) lediglich in den vier DOF der ersten Pose bestimmt, und wobei die bestimmte zweite Pose eine feste räumliche Beziehung des zweiten Trackers (16) relativ zu dem anatomischen Objekt (24) lediglich in den vier DOF der zweiten Pose bestimmt.

5. Verfahren nach einem der Ansprüche 1 bis 4, wobei
ein erster der beiden translatorischen DOF und ein erster der beiden rotatorischen DOF einer ersten Trajektorie (34; 40) zugeordnet sind, und wobei ein zweiter der beiden translatorischen DOF und ein zweiter der beiden rotatorischen DOF einer zweiten Trajektorie (36; 42) zugeordnet sind, die sich von der ersten Trajektorie unterscheidet.

6. Verfahren nach Anspruch 5, wobei
das Bestimmen der dritten Pose des virtuellen Trackers umfasst:
Bestimmen, basierend auf einer der ersten Pose und der zweiten Pose, einer dritten Trajektorie (38; 44), die durch den ersten oder zweiten Tracker (14; 16) definiert ist, der mit der ersten Pose oder der zweiten Pose assoziiert ist, wobei sich die dritte Trajektorie (38; 44) von der ersten Trajektorie (34; 40) und der zweiten Trajektorie (36; 42) unterscheidet; und
Bestimmen der dritten Pose des virtuellen Trackers ferner auf der Grundlage der dritten Trajektorie (38; 44).

7. Verfahren nach Anspruch 6, wobei
das Bestimmen der dritten Pose des virtuellen Trackers umfasst:
Bestimmen einer ersten Instanz der dritten Trajektorie (34) auf der Grundlage der ersten Pose;
Bestimmen, basierend auf der zweiten Pose, einer zweiten Instanz der dritten Trajektorie (44), wobei die zweite Instanz der dritten Trajektorie (44) relativ zu der ersten Instanz der dritten Trajektorie (34) nicht parallel ist; und
Bestimmen, basierend auf der ersten Instanz der dritten Trajektorie (34) und der zweiten Instanz der dritten Trajektorie (38), eines virtuellen Koordinatensystems (46), wobei das virtuelle Koordinatensystem (46) eine vordefinierte räumliche Beziehung relativ zu der dritten Pose des virtuellen Tracker aufweist.

8. Verfahren nach Anspruch 7, wobei
das Bestimmen des virtuellen Koordinatensystems ein Bestimmen eines ersten Richtungsvektors (48) der ersten Instanz der dritten Trajektorie (38), ein Bestimmen eines zweiten Richtungsvektors (50) der zweiten Instanz der dritten Trajektorie (44) und ein Definieren einer Koordinatenachse (52; 58) des virtuellen Koordinatensystems (46) derart, dass sie in einer von dem ersten Richtungsvektor (48) und dem zweiten Richtungsvektor (50) aufgespannten Ebene liegt, umfasst.

9. Verfahren nach Anspruch 7 oder 8, wobei
das Bestimmen des virtuellen Koordinatensystems (46) ein Definieren einer Koordinatenachse (52) des virtuellen Koordinatensystems (46) umfasst, um mit einer der ersten Instanz der dritten Trajektorie (38) und der zweiten Instanz der dritten Trajektorie (44) zusammenzufallen.

10. Verfahren nach einem der Ansprüche 7 bis 9, wobei
das Bestimmen des virtuellen Koordinatensystems (46) ein Bestimmen einer Richtung (54) eines kleinsten Abstands zwischen der ersten Instanz der dritten Trajektorie (38) und der zweiten Instanz der dritten Trajektorie (44) und ein Definieren einer Koordinatenachse (56) des virtuellen Koordinatensystems (46) derart, dass sie mit der Richtung (56) übereinstimmt, umfasst.

11. Verfahren nach Anspruch 2 oder einem der Ansprüche 3 bis 10 sofern abhängig von Anspruch 2, wobei
das Bestimmen der Transformation umfasst:
Erfassen einer Ausrichtungstrajektorie (22; 30) in den Bilddaten, wobei die Ausrichtungstrajektorie (22; 30) eine bekannte räumliche Beziehung relativ zu zumindest einem aus dem ersten Tracker (14) und dem zweiten Tracker (16) aufweist; und
Abgleichen der erfassten Ausrichtungstrajektorie (22; 30) an der Pose des zumindest einen aus dem ersten Tracker (14) und dem zweiten Tracker (16), unter Verwendung der bekannten räumlichen Beziehung.

12. Verfahren nach einem der Ansprüche 1 bis 11, wobei
zumindest einer von dem ersten Tracker (14) und dem zweiten Tracker (16) beweglich entlang einer Ausrichtungstrajektorie (22; 30) angeordnet ist, die eine feste räumliche Beziehung relativ zu dem anatomischen Objekt (24) aufweist.

13. Verfahren nach Anspruch 11 oder 12, wobei
der zumindest eine von dem ersten Tracker (14) und dem zweiten Tracker (16) mechanisch an eines oder mehrere Elemente gekoppelt ist, die ausgewählt sind aus
(i) einem linearen Element (33), das in einer festen räumlichen Beziehung relativ zu dem anatomischen Objekt (24) angeordnet ist, wobei die Ausrichtungstrajektorie (22; 30) einer Längsachse des linearen Elements (33) entspricht oder parallel dazu verläuft;
(ii) einem kanülierten Implantat (37), wobei das kanülierte Implantat (37) in einer festen räumlichen Beziehung relativ zu dem anatomischen Objekt (24) angeordnet ist, wobei die Ausrichtungstrajektorie (22; 30) einer Längsachse einer Kanülierung (39) innerhalb des kanülierten Implantats (37) entspricht oder parallel zu dieser verläuft;
(iii) einem chirurgischen Instrument (41), wobei das chirurgische Instrument (41) in einer festen räumlichen Beziehung relativ zu dem anatomischen Objekt (24) angeordnet ist, wobei die Ausrichtungstrajektorie (22; 30) einer Längsachse einer Kanülierung (43) oder eines Tracker-Behälters (47) innerhalb des chirurgischen Instruments (41) entspricht oder parallel zu dieser verläuft; und
(iv) einem linearen Loch (49) in dem anatomischen Objekt (24), wobei die Ausrichtungstrajektorie (22; 30) einer Längsachse des linearen Lochs (49) entspricht oder parallel zu dieser verläuft.

14. Verfahren nach einem der Ansprüche 1 bis 13, wobei
die ersten Tracking-Daten eine verfolgte Pose des ersten Trackers (14) in fünf oder sechs DOF angeben, und wobei das Bestimmen der ersten Pose ein selektives Vernachlässigen von einem oder zwei der DOF der verfolgten Pose des ersten Trackers (14) umfasst, um die erste Pose in den genau vier DOF zu erlangen; und/oder
wobei die zweiten Tracking-Daten eine verfolgte Pose des zweiten Trackers (16) in fünf oder sechs DOF angeben, und wobei das Bestimmen der zweiten Pose ein selektives Vernachlässigen von einem oder zwei der DOF der verfolgten Pose des zweiten Trackers (16) umfasst, um die zweite Pose in den genau vier DOF zu erlangen.

15. Verfahren nach einem der Ansprüche 1 bis 14, wobei
zumindest einer von dem ersten Tracker (14) und dem zweiten Tracker (16) ein 3-DOF-, 4-DOF- oder 5-DOF-Tracker ist, der eine Spule (32) umfasst, wobei die Spule (32) dazu konfiguriert ist, von einem elektromagnetischen Tracking-System (8) verfolgt zu werden; und/oder
zumindest einer von dem ersten Tracker (14) und dem zweiten Tracker (16) ein 3-DOF-, 4-DOF- oder 5-DOF-Tracker ist, der einen, zwei oder mehr optische Tracking-Marker (18, 20) umfasst, die eine Gerade im Raum definieren, wobei die Marker (18, 20) dazu konfiguriert sind, von einem optischen Tracking-System (6) verfolgt zu werden.

16. Verfahren nach einem der Ansprüche 1 bis 15, wobei
das anatomische Objekt (24) einen Wirbel einer Wirbelsäule (26) eines menschlichen Patienten (28) umfasst oder ein solcher ist.

17. Computersystem (100) mit zumindest einem Prozessor (2) und zumindest einem Speicher (4), wobei der zumindest eine Speicher (4) Anweisungen speichert, die bei Ausführung durch den zumindest einen Prozessor (2) den zumindest einen Prozessor (2) dazu konfigurieren:
erste Tracking-Daten für einen ersten Tracker (14) in einem Tracking-Koordinatensystem zu erlangen (300), wobei der erste Tracker (14) mit einem anatomischen Objekt (24) assoziiert ist;
basierend auf den ersten Tracking-Daten, eine erste Pose des ersten Trackers (14) in genau vier Freiheitsgraden, DOF, zu bestimmen (302), wobei die vier DOF der ersten Pose zwei translatorische DOF und zwei rotatorische DOF umfassen;
zweite Tracking-Daten für einen zweiten Tracker (16) in einem Tracking-Koordinatensystem zu erlangen (304), wobei der zweite Tracker (16) mit dem anatomischen Objekt (24) assoziiert ist;
basierend auf den zweiten Tracking-Daten, eine zweite Pose des zweiten Trackers (16) in genau vier DOF zu bestimmen (306), wobei die vier DOF der zweiten Pose zwei translatorische DOF und zwei rotatorische DOF umfassen;
basierend auf der ersten Pose und der zweiten Pose, eine dritte Pose eines virtuellen Trackers in sechs DOF zu bestimmen (308), wobei der virtuelle Tracker eine feste räumliche Beziehung relativ zu dem anatomischen Objekt (24) in den sechs DOF der dritten Pose aufweist.

18. Computersystem (100) nach Anspruch 17, das ferner dazu konfiguriert ist, das Verfahren nach einem der Ansprüche 2 bis 16 durchzuführen.

19. Computerprogrammprodukt mit Anweisungen, die bei Ausführung auf zumindest einem Prozessor (2) den zumindest einen Prozessor (2) veranlassen, das Verfahren nach einem der Ansprüche 1 bis 16 auszuführen.

20. Computerprogrammprodukt nach Anspruch 19, das auf einem oder mehreren computerlesbaren Aufzeichnungsmedien (4) gespeichert ist.

## Revendications

1. Procédé destiné à être utilisé dans la navigation chirurgicale, le procédé étant exécuté par un système informatique (100) et comprenant:
l'obtention (300) de premières données de suivi pour un premier suiveur (14) dans un système de coordonnées de suivi, dans lequel le premier suiveur (14) est associé à un objet anatomique (24);
la détermination (302), en fonction des premières données de suivi, d'une première pose du premier suiveur (14) dans exactement quatre degrés de liberté, DOF, dans lequel les quatre DOF de la première pose comprennent deux DOF de translation et deux DOF de rotation;
l'obtention (304) de secondes données de suivi pour un second suiveur (16) dans un système de coordonnées de suivi, dans lequel le second suiveur (16) est associé à l'objet anatomique (24);
la détermination (306), en fonction des secondes données de suivi, d'une seconde pose du second suiveur dans exactement quatre degrés de liberté, DOF, dans lequel les quatre DOF de la deuxième pose comprennent deux DOF de translation et deux DOF de rotation;
la détermination (308), sur la base de la première pose et de la deuxième pose, d'une troisième pose d'un suiveur virtuel dans six DOF, le suiveur virtuel ayant une relation spatiale fixe par rapport à l'objet anatomique (24) dans les six DOF de la troisième pose.

2. Procédé selon la revendication 1, comprenant en outre:
la détermination ou l'obtention d'une transformation entre (i) une quatrième pose de l'objet anatomique (24) dans six DOF dans un système de coordonnées d'image de données d'image de l'objet anatomique (24) et (ii) la troisième pose.

3. Procédé selon la revendication 2, comprenant en outre:
la détermination d'une cinquième pose de l'objet anatomique (24) dans six DOF sur la base de la troisième pose et de la transformation.

4. Procédé selon l'une quelconque des revendications à 3, dans lequel
la première pose déterminée définit une relation spatiale fixe du premier suiveur (14) par rapport à l'objet anatomique (24) dans uniquement les quatre DOF de la première pose, et dans lequel la seconde pose déterminée définit une relation spatiale fixe du second suiveur (16) par rapport à l'objet anatomique (24) dans uniquement les quatre DOF de la seconde pose.

5. Procédé selon l'une quelconque des revendications à 4, dans lequel
un premier des deux DOF de translation et un premier des deux DOF de rotation sont associés à une première trajectoire (34; 40), et dans lequel un second des deux DOF de translation et un second des deux DOF de rotation sont associés à une seconde trajectoire (36; 42) différente de la première trajectoire.

6. Procédé selon la revendication 5, dans lequel
la détermination de la troisième pose du suiveur virtuel comprend:
la détermination, sur la base de l'une parmi la première pose et la seconde pose, d'une troisième trajectoire (38; 44) définie par le premier ou le second suiveur (14; 16) associé à l'une parmi la première pose et la seconde pose, la troisième trajectoire (38; 44) étant différente de la première trajectoire (34; 40) et de la deuxième trajectoire (36; 42); et
la détermination de la troisième pose du suiveur virtuel en outre sur la base de la troisième trajectoire (38; 44).

7. Procédé selon la revendication 6, dans lequel
la détermination de la troisième pose du suiveur virtuel comprend:
la détermination, sur la base de la première pose, d'une première instance de la troisième trajectoire (34);
la détermination, sur la base de la deuxième pose, d'une seconde instance de la troisième trajectoire (44), dans lequel la seconde instance de la trajectoire (44) est non parallèle à la première instance de la troisième trajectoire (34); et
la détermination, sur la base de la première instance de la troisième trajectoire (34) et de la seconde instance de la troisième trajectoire (38), d'un système de coordonnées virtuelles (46), le système de coordonnées virtuelles (46) ayant une relation spatiale prédéfinie par rapport à la troisième pose du suiveur virtuelle.

8. Procédé selon la revendication 7, dans lequel
la détermination du système de coordonnées virtuelles comprend la détermination d'un premier vecteur directeur (48) de la première instance de la troisième trajectoire (38), la détermination d'un second vecteur directeur (50) de la seconde instance de la troisième trajectoire (44) et la définition d'un axe de coordonnées (52; 58) du système de coordonnées virtuelles (46) pour se situer dans un plan recouvert par le premier vecteur directeur (48) et par le second vecteur directeur (50).

9. Procédé selon la revendication 7 ou 8, dans lequel
la détermination du système de coordonnées virtuelles (46) comprend la définition d'un axe de coordonnées (52) du système de coordonnées virtuelles (46) pour coïncider avec l'une parmi la première instance de la troisième trajectoire (38) et la seconde instance de la troisième trajectoire (44).

10. Procédé selon l'une quelconque des revendications 7 à 9, dans lequel la détermination du système de coordonnées virtuelles (46) comprend la détermination d'une direction (54) de la plus petite distance entre la première instance de la troisième trajectoire (38) et la seconde instance de la troisième trajectoire (44), et la définition d'un axe de coordonnées (56) du système de coordonnées virtuelles (46) pour coïncider avec la direction (56).

11. Procédé selon la revendication 2 ou selon l'une quelconque des revendications 3 à 10 dans la mesure où elle dépend de la revendication 2, dans lequel la détermination de la transformation comprend:
la détection d'une trajectoire d'alignement (22; 30) dans les données d'image, la trajectoire d'alignement (22; 30) ayant une relation spatiale connue par rapport à au moins l'un parmi le premier suiveur (14) et le second suiveur (16); et
la mise en correspondance de la trajectoire d'alignement détectée (22; 30) avec la pose de l'au moins un parmi le premier suiveur (14) et le second suiveur (16), à l'aide de la relation spatiale connue.

12. Procédé selon l'une quelconque des revendications 1 à 11, dans lequel au moins l'un parmi le premier suiveur (14) et le second suiveur (16) est disposé de manière mobile le long d'une trajectoire d'alignement (22; 30) ayant une relation spatiale fixe par rapport à l'objet anatomique (24).

13. Procédé selon la revendication 11 ou 12, dans lequel
l'au moins un parmi le premier suiveur (14) et le second suiveur (16) est couplé mécaniquement à un ou plusieurs éléments choisis parmi
(i) un élément linéaire (33) disposé dans une relation spatiale fixe par rapport à l'objet anatomique (24), dans lequel la trajectoire d'alignement (22; 30) correspond à ou est parallèle à un axe longitudinal de l'élément linéaire (33);
(ii) un implant canulé (37), l'implant canulé (37) étant disposé dans une relation spatiale fixe par rapport à l'objet anatomique (24), dans lequel la trajectoire d'alignement (22; 30) correspond à ou est parallèle à un axe longitudinal d'une perforation (39) à l'intérieur de l'implant canulé (37);
(iii) un instrument chirurgical (41) l'instrument chirurgical (41) étant disposé dans une relation spatiale fixe avec l'objet anatomique (24), dans lequel la trajectoire d'alignement (22; 30) correspond à ou est parallèle à un axe longitudinal d'une canulation (43) ou à un contenant de suiveur (47) à l'intérieur de l'instrument chirurgical (41); et
(iv) un trou linéaire (49) dans l'objet anatomique (24), dans lequel la trajectoire d'alignement (22; 30) correspond à ou est parallèle à un axe longitudinal du trou linéaire (49).

14. Procédé selon l'une quelconque des revendications 1 à 13, dans lequel les premières données de suivi indiquent une pose suivie du premier suiveur (14) dans cinq ou six DOF, et dans lequel la détermination de la première pose comprend le fait d'ignorer sélectivement un ou deux des DOF de la pose suivie du premier suiveur (14) pour obtenir la première pose dans exactement quatre DOF; et/ou dans lequel les secondes données de suivi indiquent une pose suivie du second suiveur (16) dans cinq ou six DOF, et dans lequel la détermination de la seconde pose comprend le fait d'ignorer sélectivement un ou deux des DOF de la pose suivie du second suiveur (16) pour obtenir la seconde pose dans exactement quatre DOF.

15. Procédé selon l'une quelconque des revendications 1 à 14, dans lequel au moins l'un parmi le premier suiveur (14) et le second suiveur (16) est un suiveur 3-DOF, 4-DOF ou 5-DOF comprenant une bobine (32), dans lequel la bobine (32) est conçue pour être suivie par un système de suivi électromagnétique (8); et/ou au moins l'un parmi le premier suiveur (14) et le second suiveur (16) est un suiveur 3-DOF, 4-DOF ou 5-DOF comprenant un, deux, trois ou plusieurs marqueurs de suivi (18, 20) définissant une ligne dans l'espace, dans lequel les marqueurs (18, 20) sont configurés pour être suivis par un système de suivi optique (6).

16. Procédé selon l'une quelconque des revendications 1 à 15, dans lequel l'objet anatomique (24) comprend ou est une vertèbre d'une colonne vertébrale (26) d'un patient humain (28).

17. Système informatique (100) comprenant au moins un processeur (2) et au moins une mémoire (4), l'au moins une mémoire (4) stockant des instructions qui, lorsqu'elles sont exécutées par l'au moins un processeur (2), configurent l'au moins un processeur (2) pour:
obtenir (300) des premières données de suivi pour un premier suiveur (14) dans un système de coordonnées de suivi, dans lequel le premier suiveur (14) est associé à un objet anatomique (24);
déterminer (302), en fonction des premières données de suivi, une première pose du premier suiveur (14) dans exactement quatre degrés de liberté, DOF, dans lequel les quatre DOF de la première pose comprennent deux DOF de translation et deux DOF de rotation;
obtenir (304) des secondes données de suivi pour un second suiveur (16) dans un système de coordonnées de suivi, dans lequel le second suiveur (16) est associé à l'objet anatomique (24);
déterminer (306), en fonction des secondes données de suivi, une seconde pose du second suiveur dans exactement quatre degrés de liberté, DOF, dans lequel les quatre DOF de la seconde pose comprennent deux DOF de translation et deux DOF de rotation;
déterminer (308), sur la base de la première pose et de la seconde pose, d'une troisième pose d'un suiveur virtuel dans six DOF, le suiveur virtuel ayant une relation spatiale fixe par rapport à l'objet anatomique (24) dans les six DOF de la troisième pose.

18. Système informatique (100) selon la revendication 17, configuré en outre pour exécuter le procédé selon l'une quelconque des revendications 2 à 16.

19. Produit programme informatique comprenant des instructions qui, lorsqu'elles sont exécutées par au moins un processeur (2), amènent l'au moins un processeur (2) à exécuter le procédé selon l'une quelconque des revendications 1 à 16.

20. Produit programme informatique selon la revendication 19, stocké sur un ou plusieurs supports d'enregistrement lisibles par ordinateur (4).
